(19) Europäisches Patentamt / European Patent Office / Office européen des brevets

(11) **EP 4 706 538 A1**

(12) **EUROPEAN PATENT APPLICATION**

(43) Date of publication:
**11.03.2026 Bulletin 2026/11**

(21) Application number: **24198491.3**

(22) Date of filing: **04.09.2024**

(51) International Patent Classification (IPC):
**A61B 5/257** (2021.01)   **A61B 5/28** (2021.01)
**A61B 5/00** (2006.01)

(52) Cooperative Patent Classification (CPC):
**A61B 5/6833; A61B 5/257; A61B 5/28;**
A61B 5/259; A61B 2562/125

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **GELISSEN, Jozef Hubertus**
**Eindhoven (NL)**
• **WELLINGTON, Traci Adelle**
**Eindhoven (NL)**

(74) Representative: **Philips Intellectual Property & Standards**
**High Tech Campus 52**
**5656 AG Eindhoven (NL)**

(54) **ELECTRODES FOR PHYSIOLOGICAL MEASUREMENTS**

(57) An electrode (1) for physiological measurements is provided. The electrode comprises a conductive element (10) for acquiring a biosignal from a subject, a connector (20) for connecting to a biosignal acquisition device, e.g., a wearable patch, patient monitor, etc; an adhesive layer (30) for adhering the electrode to the subject and a cutting line (40, 40a, 40b). The adhesive layer defines a first outer edge (35) of a first surface (36) of the electrode (1) and the cutting line at least partly defines a second outer edge (45) of a second surface (46) of the electrode. The second surface (46) is a subsection of the first surface (36) and the second surface (46) is arranged to transfer the biosignal from the subject via the conductive element to the snap connector. The cutting line can be used to guide a user to trim the electrode into a reduced size and/ or a different shape. A device (2) for physiological measurements adapted to trim an electrode (1, 3) along a cutting line is also provided.

FIG. 2

EP 4 706 538 A1

**Description**

FIELD OF THE INVENTION

The invention relates to an electrode for physiological measurements and a device for trimming an electrode for physiological measurements.

BACKGROUND OF THE INVENTION

Around 1900, Willem van Einthoven invented the first electrocardiograph using buckets of saltwater to act as electrodes. Nowadays an electrode can be a small adhesive plaster with a high viscous salt-solution in the form of a gel and a mechanical/electrical connection to a lead-wire cable running to an ECG device, e.g., patient monitor.
More generally, electrodes are conductive pads adapted to be attached to the body surface of a subject and are used to connect a biosignal acquisition device with the body. Electrodes are components for acquiring electrical signals from the body surface. Any pair of electrodes can measure the electrical potential difference between the two corresponding locations of attachment. Electrodes can be used for electrophysiological measurements, such as electroencephalography (EEG), electrocardiography (ECG) and electromyography (EMG).
There are different types of electrodes for physiological measurements, depending on their design, material, and application. One common classification is based on the use of conductive gel or paste between the electrode and the skin, which can enhance electrical contact and reduce impedance and noise. These electrodes are called hydrogel or wet electrodes. Another type of electrodes for physiological measurements is dry electrodes, which do not require any gel or paste and can be directly attached to the skin. Both wet and dry electrodes are typically made of flexible materials, such as polymers, textiles, or rubber, that can conform to the skin surface.
There are also different mechanical-electrical connections between an electrode and a biosignal acquisition device, e.g., a measurement device, a wearable patch, and a patient monitor. The most common one is the snap connector, which is a small stubble at a side of the electrode acting as a common, standardized interface to either a snapper or grabber adapter, which either directly or through a lead-wire connects the subject to a biosignal acquisition device. An advantage of snap electrodes is that they are cheap and well accepted in the medical field due to the standardized interface. There are also pre-wired electrodes, where a lead-wire runs from the electrode to a standardized medical connector. These electrodes provide a more secure connection and reduce pressure points on the subject compared to snap electrodes. Pre-wired electrodes are mainly used in long term monitoring and on frail patients, e.g., neonatal patients. Furthermore, there are tab electrodes, which require a clamp-like adapter. Tab electrodes are the most inexpensive of the three types of electrodes and are mainly used for very short electrophysiological assessments (resting ECG).
Electrodes for physiological measurements also vary in their size and shape, depending on the target organ or tissue, the size of the subject and the measurement technique. For example, neonatal electrodes, e.g., electrodes used for baby ECG monitoring are smaller in size compared to electrodes for ECG monitoring of adults. Smaller electrode size for neonatal monitoring is required as the area of the baby chest is generally in competition with other factors, such as chest tubes, PEG tubes, wound dressings, etc. However, smaller electrodes may also be relevant for adults as well. For example, for post-cardiothoracic surgery in adults, ECG electrode placement is often adjusted or overlapped according to the available space, thus using smaller electrodes may be advantageous. Clinical implications with adjusted placement of electrodes may include shift in axis and can compromise ECG amplitude depending on placement, inconsistency with 12 lead mapping for muscle wall changes and issues with detection of post-surgery ischemia.

SUMMARY OF THE INVENTION

In view of the above, different sizes and shapes of electrodes are required to accommodate the different monitoring needs. Thus, healthcare institutions as well as their suppliers need to keep in stock electrodes of different sizes and/or shapes. Furthermore, nurses may not always have the proper electrode available when it is needed.
It is, inter alia, an object of the invention to provide an electrode for physiological measurements and a device for trimming an electrode for physiological measurements, enabling the reduction of the variety in size and shape of electrodes to manufacture and to maintain in inventory.
The invention is defined by the independent claims. Advantageous embodiments are defined in the dependent claims.
According to a first aspect of the invention, an electrode for physiological measurements is provided, the electrode comprising:

> a conductive element for acquiring a biosignal from a subject;

a connector for connecting to a biosignal acquisition device;
an adhesive layer for adhering the electrode to the subject; and
a cutting line;
wherein the adhesive layer defines a first outer edge of a first surface of the electrode;
wherein the cutting line at least partly defines a second outer edge of a second surface of the electrode;
wherein the second surface is a subsection of the first surface; and
wherein the second surface is arranged to transfer the biosignal from the subject via the conductive element to the connector.

According to the invention, the electrode comprises a cutting line, i.e., a trimming line, whose purpose is to guide the user to trim the electrode to a smaller size and/or to a different shape. An advantage thereof is that it enables manufacturers to reduce the diversity in size and shape of electrodes for production. For example, instead of manufacturing different stock-keeping units (SKU) of electrodes having the same constructional elements, where their difference lies in a different size or shape, manufacturers can manufacture fewer SKUs, e.g., one SKU, having a cutting line, so that it can guide the user to trim the electrode to a size and/or shape according to the needs of the specific subjects to be monitored. Such a reduction in manufacturing of electrode SKUs may reduce complexity in manufacturing of electrodes and may thus result in cost reductions in manufacturing of electrodes.

Accordingly, the invention enables suppliers and healthcare institutions to keep in stock fewer different SKUs of electrodes, and larger amounts of the fewer SKUs. Therefore, cost reductions may also be achieved for suppliers and healthcare institutions. Furthermore, by keeping a larger stock of fewer different SKUs of electrodes, may render them less vulnerable to any supply chain disruptions.

Another advantage is that the workflow of a user may also be improved. For example, a nurse may only need to have in hand electrodes of one larger size. Instead of losing valuable time to find a proper electrode for the occasion, they can trim the electrodes according to their needs, based on the provided cutting line.

The electrode is for example a dry electrode or a wet electrode.

In an example, the adhesive layer for adhering the electrode to the subject is fully or partly made of acrylic, silicone or rubber.

In an example, the conductive element is at least partly adhesive itself. According to this example, the adhesive layer comprises a first adhesive layer part which at least partly surrounds the conductive element and a second adhesive layer part, which is the adhesive layer of the conductive element.

In an example, the conductive element is partly or fully surrounded by the adhesive layer.

In an example, the conductive element protrudes from the adhesive layer.

In another example, the conductive element is positioned within the adhesive layer.

In another example, the conductive element is partly positioned within the adhesive layer and partly protrudes from the adhesive layer.

In an example, the connector connects both mechanically and electrically to the biosignal acquisition device.

In an example, the connector is a snap connector. In an example the connector is a lead-wire. In an example the connector is a tab.

In an example, the connector is electrically connected to the conductive element.

In an example, the first outer edge is the outer edge of the electrode. In an example, the first outer edge does not coincide with the perimeter of the electrode.

In an example, the second outer edge is partly defined by the cutting line and partly by the first outer edge.

In an example, the conductive element is fully located within the second surface. In an example, the conductive element is located between the first and the second surface.

In an embodiment, the electrode comprises two or more different cutting lines.

Having two or more cutting lines on the electrode the user may be presented with more than one options to trim the electrode to a desired size and/or shape. Thus, providing more flexibility and accommodating to an increased number of different potential shapes and sizes of a trimmed electrode.

In an embodiment, the electrode has a first side, and a second side opposing the first side, the first side comprising the adhesive layer, and the cutting line is located at the first and/or at the second side.

In an example, the first side is adapted to be adhered to the subject, e.g., the first side is adapted to be in contact with the skin of the subject. In an example, the second side is the side adapted to face away from the skin, when the electrode is worn.

For example, the cutting line is located at the first side, at the second side, or at both the first side and the second side. Furthermore, having the same cutting line located on both sides may improve ease of use and workflow. The user may start trimming the electrode holding it at one side and in case they need to stop trimming to tend to the subject, e.g., tend to an emergency, they can resume trimming without having to check which side comprises the cutting line. Furthermore, in case the electrode comprises more than one different cutting lines, the first side may comprise different cutting lines than the second side. Thus, fewer cutting lines per side can be achieved for the same number of cutting lines provided on the electrode, thus ease of use is improved, as fewer cutting lines at the same side may be easier for a user to discern.

In an embodiment, the cutting line is located on a template layer, and the template layer is attached to the electrode.

A template layer may be an extra layer, e.g., a piece of paper or foil which is attached e.g., adhered to the electrode. The template layer comprises the cutting line. Such a template may simplify the manufacturing process of electrodes comprising cutting lines, as the existing equipment and processes for electrodes without cutting lines can be used, and in a final step, a template comprising the cutting line can be attached on the existing electrodes. The template layer can be attached on the first side, on the second side or both on the first and on the second side of the electrode.

In an embodiment, the template layer is permanently attached to the electrode, i.e., a user cannot detach the template layer from the electrode.

In an embodiment, the template layer is a removable template layer, i.e., the removable template layer is adapted to be detached from the electrode. An advantage of the removable template layer is that it allows users to detach the layer comprising the cutting line before using the trimmed electrode with the subject. Thus, the electrode used on the subject does not include any ink from the cutting line or parts of the cutting line which were not trimmed and contact between the ink and the subject's skin is avoided, e.g., due to leaching from sweat. A removable template layer also enhances the usability of the electrode. The user may remove the template layer comprising the cutting line, before trimming the electrode and can then position the template layer on the designated attachment area on the subject. Based on this, the user can get a better perspective of the shape and extent to which the electrode needs to be trimmed for the specific subject. Subsequently, the user can reapply the template to the electrode and trim the electrode accordingly.

In an embodiment, the electrode comprises a protective layer for protecting the adhesive layer. In an embodiment, the template layer is adhered on the protective layer.

In an embodiment, the template layer includes the protective layer. Thus, the cost of manufacturing is reduced and fewer materials are used for manufacturing, as the template layer and the protective layer are combined in one single layer.

In an embodiment, the cutting line outlines the shape of at least one of a circle, an ellipse, a hexagon and a square.

An advantage of this embodiment is that the user may not only trim the electrode to a smaller size but may also choose to change its shape. For example, the initial shape of the electrode is that of a circle and the user by trimming the electrode may change its shape to an ellipse, a hexagon or a square. The square may for example be a rounded square and, in another example, a squircle. Such a change in the shape of the electrode is desired in cases where the position of the electrode on the subject's body is close to tubes, wound dressings etc. Furthermore, the electrode covering areas of the subject that easily fold or skin area that easily moves, e.g., towards the arms should be avoided, as it may cause skin irritation and early detachment. Changing the shape of the electrode may help to avoid these drawbacks.

Preferably, the cutting line outlines a shape having rounded edges. Rounded edges have been proven to cause fewer failure points compared to pointy edges. Failure points are areas of the electrode that begin to peel away from the skin. As they remain tacky, they can adhere to clothing or other surfaces when rubbed, causing the initial detachment from the skin to expand further.

According to a second aspect of the invention, a device adapted to trim an electrode for physiological measurements is provided, wherein the electrode comprises:

  a conductive element for acquiring a biosignal from a subject;
  a connector for connecting to a biosignal acquisition device; and
  an adhesive layer for adhering the electrode to the subject, wherein the adhesive layer defines a first outer edge of a first surface of the electrode;

the device comprising:

  a holder for holding the electrode, and;
  a trimming mechanism adapted to trim the electrode along a cutting line;
  wherein the cutting line at least partly defines a second outer edge of a second surface of the electrode;
  wherein the second surface is a subsection of the first surface; and
  wherein the second surface is arranged to transfer the biosignal from the subject via the conductive element to the connector.

An advantage of the proposed solution is that the user may not need to use scissors, or a safety cutter to trim the electrode to a smaller size and/or a different shape. A user having a device according to this aspect may trim electrodes which may not comprise a cutting line, as the final size and/or shape of the electrode is defined by the trimming mechanism.

Using such a device may also improve ease of use and workflow for the user, as electrodes may be trimmed quicker and with less effort than using e.g., scissors.

In an embodiment, the trimming mechanism is configurable to trim the electrode along two or more different cutting lines. According to this embodiment, the device thus comprises a mechanism enabling the user to configure the trimming mechanism to trim an electrode to more than one size or shapes. Thus, it provides more flexibility and accommodates to an increased number of different potential shapes and sizes of a trimmed electrode.

In an embodiment, the device is adapted to trim the electrode along a cutting line, wherein the cutting line outlines the shape of at least one of a circle, an ellipse, a hexagon and a square.

As discussed in more detail above, it may be advantageous to change the shape of the electrode in view of the specific needs for the subject.

In an embodiment, the device is adapted to trim an electrode as defined by the first aspect of the invention. Thus, the device may be used both for electrodes comprising and not comprising a cutting line.

In an embodiment the holder comprises the trimming mechanism. Therefore, the cutting mechanism and the holder might be integrated into a single structure. Consequently, this could simplify the building process of the device by utilizing a singular element that serves as both the holder and the cutter.

These and other aspects of the invention will be apparent from and elucidated with reference to the embodiments described hereinafter.

BRIEF DESCRIPTION OF THE DRAWINGS

Fig. 1 shows a section of an electrode for physiological measurements according to an embodiment of the invention.
Fig. 2 shows a first side of an electrode for physiological measurements according to an embodiment of the invention.
Fig. 3 shows a second side of an electrode for physiological measurements according to an embodiment of the invention.
Fig. 4 shows a first side of an electrode for physiological measurements according to an embodiment of the invention.
Fig. 5 shows a device adapted to trim an electrode for physiological measurements according to an embodiment of the invention.
Fig. 6 shows a device adapted to trim an electrode for physiological measurements according to another embodiment of the invention.
Fig. 7 shows a holder of a device adapted to trim an electrode for physiological measurements and an electrode, according to an embodiment of the invention.

DESCRIPTION OF EMBODIMENTS

The invention will be described with reference to the Figures. The detailed description and specific examples, while indicating exemplary embodiments of the invention, are intended for purposes of illustration only and are not intended to limit the scope of the invention. These and other features, aspects, and advantages of the present invention will become better understood from the following description, appended claims, and accompanying drawings. The Figures are merely schematic and are not drawn to scale. It should also be understood that the same reference numerals are used throughout the Figures to indicate the same or similar parts.

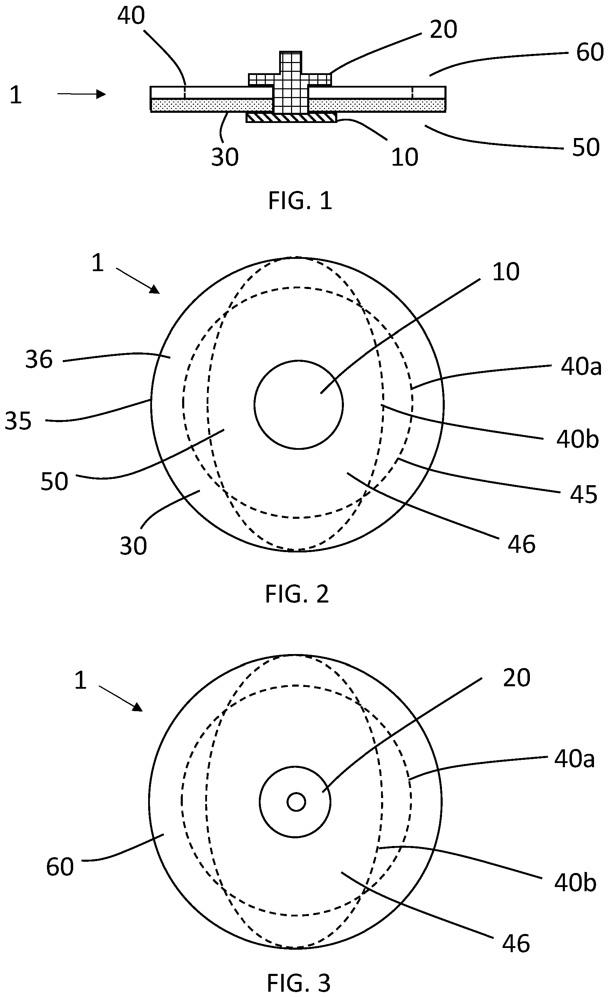

In a first aspect of the invention, an electrode for physiological measurements is provided. The electrode is for example a dry electrode or a wet electrode. Figs. 1-4 show examples of an electrode 1 according to embodiments of the invention. The electrode 1 comprises a conductive element 10 for acquiring a biosignal from a subject. Any suitable conductive element may be used. In an example, the conductive element 10 of a wet electrode is solid gel or a wet gel. In case a wet gel is used, a sponge structure may be included. In an embodiment where the electrode is a dry electrode, the conductive element 10 may comprise Ag/AgCl conductive plastic.

The electrode 1 further comprises an adhesive layer 30 for adhering the electrode to the subject. Any suitable known adhesive may be used. In embodiments according to the invention, the conductive element 10 is at least partly adhesive itself. According to these embodiments, the adhesive layer 30 comprises two parts: a first adhesive layer part which at least partly surrounds the conductive element 10, and for example is made of acrylic, silicone or rubber and a second adhesive layer part, which is the adhesive layer of the conductive element. In the embodiments as shown in Figs 1, 2 and 4, the conductive element 10 is fully surrounded by the adhesive layer 30. In another example, the conductive element 10 is partly surrounded by the adhesive layer. In the embodiment shown in Fig. 1 the conductive element 10 is exemplary shown as a layer protruding from the adhesive layer 30 e.g., is positioned upon the adhesive layer 30. In another example, the conductive element 10 is a layer positioned within the adhesive layer 30. For example, the first adhesive layer part forms a ring and the conductive element is positioned within this ring. In another example, the conductive element is partly positioned within the adhesive layer 30 and partly protrudes from the adhesive layer 30. In the embodiment where the conductive element 10 protrudes from, e.g., extends beyond the adhesive layer 30, it is to be understood that it is still at least partly surrounded by the adhesive layer, e.g., as seen from a top or bottom view of the electrode (Figs 2-4).

The electrode 1 comprises two sides. The first side 50 is the layer adapted to be adhered on the subject, e.g., the side to be in contact with the skin. The first side 50 thus comprises the adhesive layer 30. The second side 60 of electrode 1 is the side opposing the first side 50, i.e., the side adapted to face away from the skin, when the electrode is worn. The second side typically has a soft surface, e.g., non-woven cloth or foam.

In an embodiment, the conductive element 10 covers the whole first side 50. Thus, the whole side of the electrode adapted to be in contact with the skin of the subject is conductive. In this embodiment, the adhesive layer 30 consists of the adhesive layer of the conductive element 10.

In an embodiment, the electrode 1 comprises a carrier material (not shown in the Figures), which is used in an extra layer, having the role of a foundation on which the components of the electrode are held together. The carrier material is typically made of foam, cloth, or silicone. The carrier material at the second side 60 of the electrode, i.e., the side adapted to face away from the skin of the subject when the electrode is worn, typically has a soft surface, e.g., comprises non-woven cloth or foam. Alternatively, the electrode 1 does not comprise such a carrier material. An electrode without a carrier material may comprise an adhesive layer 30 which on the second side 60 has a soft surface.

The electrode 1 comprises a connector 20 for connecting to a biosignal acquisition device, e.g., a patient monitor, a wearable patch, etc. The connector connects both mechanically and electrically to the biosignal acquisition device. The biosignal acquisition device may process the acquired biosignal and produce an output, e.g., ECG, EEG, etc. The connector 20 shown in Figs 1 and 3 is a snap connector, e.g., a standardized interface to either a snapper or grabber. In another embodiment according to the invention, not shown in the Figures, connector 20 is a lead-wire having an interface suitable for connecting to a biosignal acquisition device. Such a connector is used e.g., at pre-wired electrodes. In another embodiment according to the invention, connector 20 is a tab suitable for connecting to a biosignal acquisition device using a clamp-like mechanism. Such a connector is used e.g., at tab electrodes. The connector 20 is electrically connected to the conductive element 10 and comprises the interface between the electrode and the biosignal acquisition device. According to the invention, any suitable known connector may be used.

According to the invention, the adhesive layer 30 defines a first outer edge 35 of a first surface 36 of the electrode. In the embodiments shown in Figs 1 - 4, the diameter of the adhesive layer 30 equals the diameter of the electrode 1. In these embodiments the first outer edge 35 is the outer edge of the electrode. In other embodiments, e.g., wherein the electrode also comprises a carrier material, the surface of the carrier material may be larger than the surface of the adhesive layer. For example, the electrode at its first side 50, i.e., the side adapted to adhere to the subject, may have areas which are not adhesive. In such embodiments the first outer edge 35 does not coincide with the outer perimeter of the electrode.

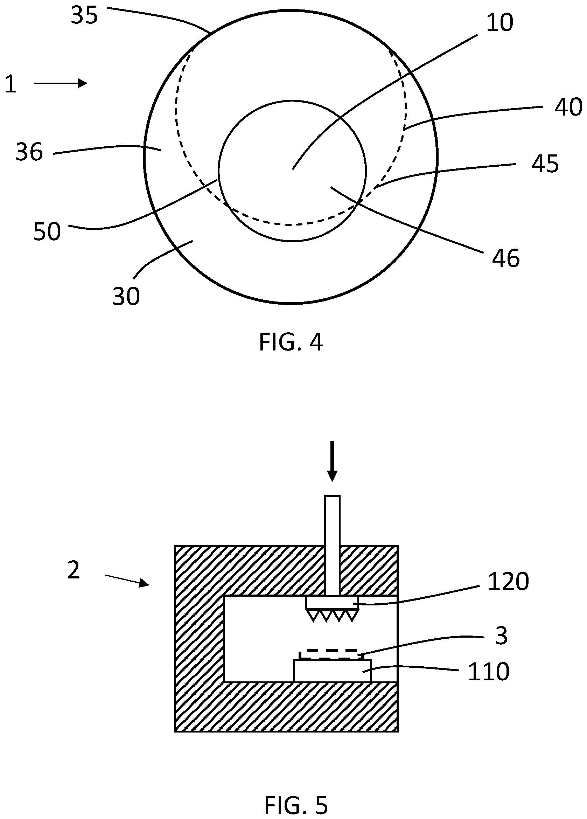

The electrode 1 further comprises a cutting line 40, 40a, 40b, which is a marking based on which the user may trim the electrode 1. The cutting lines 40 (Fig. 4) and 40a (Figs 2, 3) are exemplary circular. In Figs 2 and 3 the cutting line 40b is elliptical. The cutting line may outline any shape. In an example the cutting line outlines a square including a rounded square, a squircle, a star, a rectangular etc. Preferably, the cutting line 40, 40a, 40b outlines a shape having rounded edges.

The cutting line 40, 40a, 40b, at least partly defines a second outer edge 45 of a second surface 46 of the electrode. The second outer edge 45 may be considered as a guideline defining a boundary up to which a user may trim the electrode. For example, the user may trim the electrode 1 along the cutting line 40, 40a, 40b. For example, the user guided by the cutting line 40, 40a, 40b may trim the electrode preferably within the surface defined between the first outer edge 35 and the second outer edge 45. The second surface 46 is a subsection of the first surface 36, i.e., the second surface 46 covers an area smaller than the first surface 36. The cutting line 40, 40a, 40b can be located at the first side 50 as shown in Fig. 2, at the second side 60 as shown in Fig. 3 or both at the first side 50 and at the second side 60.

The first 36 and second 46 surfaces are defined by a first 35 and second 45 outer edge accordingly. The edges can be considered that they extend across both the first 50 and the second 60 side of the electrode 1. Thus, in an embodiment where the cutting line 40, 40a, 40b is located at the second side 60 of the electrode 1, the second outer edge 45 defines the second surface 46 also at the first side 50.

The cutting line 40, 40a, 40b may partly define the second outer edge 45. For example, the second outer edge 45 may be partly defined by the cutting line 40 and partly by the first outer edge 35, as shown in Fig. 4. Although Fig. 4 shows the first side 50 of the electrode 1, the cutting line 40, 40a, 40b according to this embodiment may also be located at the second side 60 of the electrode 1.

The second surface 46, i.e., the surface having a perimeter defined by the second outer edge 45, is arranged to transfer the biosignal from the subject via the conductive element 10 to the connector 20. Thus, the conductive element 10, which is arranged to acquire the biosignal, is at least partly located within the second surface 46. In an embodiment, the conductive element 10 is partly located between the first and partly between the second surface 46 as exemplary shown in Fig. 4. This means that the cutting line 40, 40a, 40b passes through the surface of the conductive element 10 and that the conductive element 10 may be partly trimmed. In another embodiment, the cutting line does not cross the conductive element 10, thus the conductive element 10 remains intact in the trimmed electrode.

In an embodiment, the surface of the conductive element 10 equals the surface of the first surface 36, i.e., the whole first side 50 is covered by the conductive element 10, wherein the conductive element 10 also comprises the adhesive layer 30. In this embodiment, when trimming the electrode 1, the conductive element 10 is partly trimmed.

The cutting line 40, 40a, 40b may be directly printed, engraved, or placed using any known technology on the electrode 1. In an embodiment, the cutting line 40, 40a, 40b is placed, e.g., printed, on a separate template layer and then the template layer is attached, e.g., adhered on the electrode 1. In an embodiment, the template layer preferably fully covers the first 50 and/or second 60 side of the electrode 1. Additionally, or alternatively, the cutting line 40, 40a, 40b is placed on the adhesive layer 30. Additionally, or alternatively, the cutting line 40, 40a, 40b is placed on the carrier material.

In an example, the first side 50, i.e., the side comprising the adhesive layer 30 and which is adapted to be adhered to the subject, comprises a protective layer for the adhesive layer 30 which the user removes before applying the electrode to the subject. The purpose of the protective layer is to prevent dirt, dust, and other contaminants from sticking to the adhesive material of the adhesive layer 30. In electrodes, this is usually a transparent plastic. In an example for dry electrodes such a protective layer is siliconized paper. In an embodiment, the template layer includes the protective layer. In an embodiment, the template layer is adhered on the protective layer.

In an embodiment, the electrode 1 comprises two or more different cutting lines 40, 40a, 40b, as also shown in Figs 2 and 3. Thus, the electrode may provide more than one solutions to a user. In an embodiment the electrode 1 provides a cutting line at side 50 and another cutting line at side 60 which outlines a different shape and/or has different dimensions than the cutting line at side 50.

In a second aspect of the invention, a device 2 adapted to trim an electrode 1, 3 for physiological measurements is provided. For example, the device 2 is adapted to trim the electrode 1 according to the first aspect of the invention. For example, the device 2 is adapted to trim an electrode 3 that does not have a cutting line. For example, the electrode 3 is the same as the electrode according to the first aspect, except that the electrode 3 does not have the cutting line.

Embodiments of such a device are shown in Figs. 5-7. The device 2 comprises a holder 110, where the electrode 1,3 can be positioned. In an example, the holder is a cavity, where the user can insert the electrode 1,3. In another example the holder is a surface, e.g., a disk or a ring, on top of which the user may place the electrode 1,3. Any holder suitable to hold an electrode may be used. Fig. 5-7 show an electrode 3 positioned on holder 110. The electrode to be used with this device 2 may be any electrode 3 which does not comprise a cutting line, or an electrode 1 comprising a cutting line 40, 40a, 40b.

The device also comprises a trimming mechanism 120 for trimming the electrode 1, 3 along a cutting line 40, 40a, 40b. The trimming mechanism 120 may comprise one or more blades, or it may be any other structure which is suitable to trim an electrode. In the second aspect of the invention, the cutting line is thus defined by the trimming mechanism 120, while the cutting line according to the first aspect is a marking on the electrode 1.

The device 2 may be manually or electrically operated. The user places the electrode 1, 3 in or on the holder, actuates the device 2 and the trimming mechanism 120 trims the electrode 1,3 along a cutting line 40, 40a, 40b.

Fig. 5 shows an embodiment of device 2, with the holder 110 on which the electrode 1, 3 can be positioned and the trimming mechanism 120. According to this embodiment, when the device is operated, the trimming mechanism 120 is pushed (as exemplary shown by the arrow) towards the direction of the holder 110. The trimming mechanism 120 then trims the electrode 1, 3 along the cutting line 40, 40a, 40b.

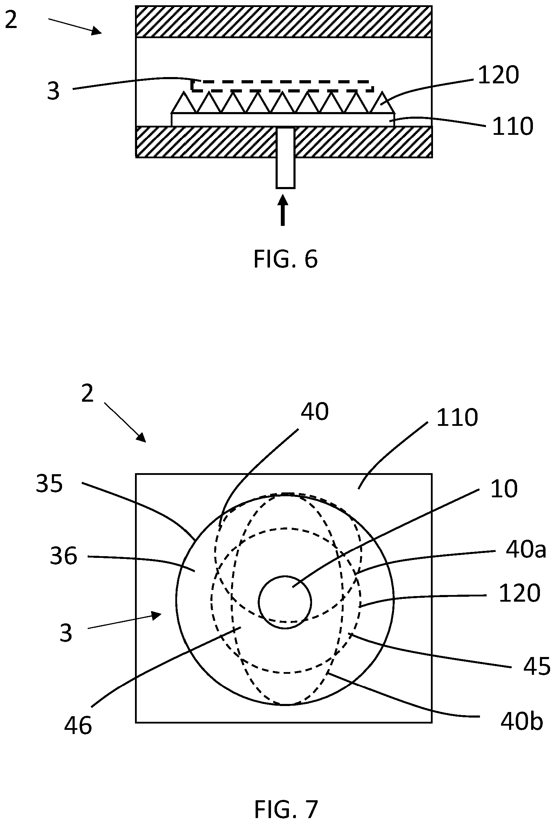

Fig. 6 shows another embodiment according to the invention, wherein the holder 110 also comprises the trimming mechanism 120. Thus, essentially the trimming mechanism 120 may also be the holder 110 where the electrode 1, 3 is positioned. As shown in Fig. 6, the trimming mechanism 120 holding the electrode 1, 3 is pushed towards a wall within device 2 and the electrode 1, 3 can be trimmed accordingly.

Fig. 7 shows an electrode 3 positioned on a holder 110. The electrode 3 does not comprise a cutting line. The cutting line 40, 40a, 40b is defined i.e., outlined, by trimming mechanism 120, e.g., by the blades or edges of the trimming mechanism 120. As already discussed herein, the adhesive layer 30 defines a first outer edge 35 of a first surface 36 of the electrode and the cutting line 40, 40a, 40b (although not part of the electrode 3), at least partly defines a second outer edge 45 of a second surface 46 of the electrode 3, the second surface 46 is a subsection of the first surface 36, and the second surface 46 is arranged to transfer the biosignal from the subject via the conductive element 10 to the connector. As a result, the device 2 is used to trim an electrode resulting in a trimmed electrode having smaller dimensions and/or a different shape than before it was trimmed.

Features already discussed regarding the first aspect of the invention may correspond accordingly to the second aspect of the invention and thus will not be discussed again. For example, the features, examples and embodiments regarding the adhesive layer 30, conductive element 10, cutting line 40, 40a, 40b, first 35 and second 45 outer edges, and first 36 and second 46 surfaces that have already been discussed regarding the first aspect of the invention, may also apply accordingly to the second aspect of the invention.

In an embodiment according to the invention, the electrode 1,3 at the second surface 46 fulfills the requirements of ANSI/AAMI EC12 standard. Therefore, the trimmed electrode also fulfills the requirements of the standard.

In an embodiment according to the invention, the first surface 36 of the electrode 1, 3 has the dimensions and/or shape of an electrode adapted for adult monitoring and the cutting line 40, 40a, 40b defines a second outer edge 45 of a second surface 46 of the electrode, wherein the second surface 46 has the dimensions and/or shape of an electrode adapted for neonatal monitoring. Thus, the electrode 1,3 is an electrode used for adult monitoring and the user may trim it to reduce its size to an electrode to be used for neonatal monitoring.

In an embodiment according to the invention, the adhesive layer 30 at the second surface 46 of the electrode 1,3 provides adhesion force higher than the sum of external forces which may be applied during use of the trimmed electrode 1,3. Thus, adherence of the trimmed electrode 1,3 on the skin and the acquired biosignal quality may be improved. In an example regarding external forces of snap electrodes, if the attached lead-wire is tugged, the adhesion force of the trimmed electrode 1,3 should cause the lead-wire to disengage from the electrode and the electrode should remain attached to the skin. In an example of a trimmed electrode 1,3 attached to a wearable patch, the electrode 1,3 should be able to carry the weight of the patch. The adhesion force required for a subject to carry a wearable patch for a certain number of days is different for example to the adhesion force requirement for a neonatal patient connected with pre-wired electrodes. In an example of wet electrodes, the conductive gel of the electrode 1,3 may swell because of absorbing external moisture e.g., sweat, water. Such swelling may increase the volume of the gel pushing the attached electrode 1,3 away from the skin and creating a force opposite to the adhesion force.

The adhesion force of the adhesive layer 30 depends on the adhesion characteristics of the skin adhesive, e.g., acrylic, silicone, rubber, etc. As is generally known, the adhesion force is also related to the peel force, i.e., the amount of force needed to separate two bonded materials by peeling one away from the other.

For wet and dry electrodes, the inventors propose different equations for calculating the area covered by the second surface 46, i.e., the surface area defined by the cutting line 40, 40a, 40b, which may also be the total surface of an electrode 1,3 trimmed based on the cutting line 40, 40a, 40b. One main reason is that the conductive element 10 of dry electrodes may be adhesive as well, while for wet electrodes the gel does not exhibit substantial adhesive properties. By calculating the area covered by the second surface 46, its perimeter may also be derived, thus also the perimeter of the second outer edge 45.

For wet electrodes the inventors propose the following equation:

$$\text{Required adhesion force}_{wet} = \text{Peel force} \times (\text{second surface area} - \text{non adhered surface area}) \tag{1},$$

Wherein the required adhesion force is the target adhesion force of adhesive layer 30 at the second surface 46 and Peel force is the peel force of the adhesive material of the adhesive layer 30. Non adhered surface area is the area comprising the wet gel with the conductive element 10. The minimum size of the non adhered surface area is the surface area covered by the conductive element within the second surface 46. Nevertheless, taking into account swelling of the gel, this surface area may increase. For the calculations its size can be chosen based on the type and dimensions of the gel, and the estimated swelling of the gel.

Thus, the area of the second surface 46 can be estimated according to equation (1). The required adhesion force$_{wet}$ is chosen based on the use case of the electrode, for example as discussed above. The peel force depends on the adhesive material of the adhesive layer 30.

The peel force may be calculated in relation to test materials different to skin, e.g., Polyethylene, steel, etc. Examples of peel force of materials used as skin adhesives can be 7- 15 N / 25.4 mm for adhesion to Low Density Polyethylene and 2 N / 24 mm for adhesion on skin.

The peel force may be provided as force per width of the material (instead of force per surface area), due to the techniques used for peel strength testing, wherein the materials are peeled apart at a constant speed and the strength of the force needed to separate the materials is calculated along with the width of the bond surface to determine the peel strength.

Thus, equation (2) can also be used for wet electrodes:

$$\text{Required adhesion force}_{wet} = \text{Peel force} \times (\text{max width}_{\text{second surface area}} - \text{max width}_{\text{non adhered}} \text{ surface area}) \tag{2},$$

Wherein the required adhesion force is the target adhesion force of adhesive layer 30 at the second surface 46, Peel force is the peel force of the adhesive material of the adhesive layer 30, max width$_{\text{second surface area}}$ is the maximum width of the second surface 46, at a direction perpendicular to the direction of the peel force, e.g., for a circular electrode it is the diameter of the second surface 46, and for a square electrode it is its side. Max width$_{\text{non adhered surface area}}$ is the maximum width of the non adhered area, at a direction perpendicular to the direction of the peel force, e.g., for a circular gel it is the diameter of the gel.

By calculating the maximum width of the second surface 46, the area of the second surface 46 can be derived.

For dry electrodes account should be taken on the fact that the materials comprising the conductive element 10 may exhibit adhesive properties. In an example, the material of the conductive element 10 is a skin adhesive, which is made conductive by the addition of materials having electric conductivity.

For calculating the second surface 46 of dry electrodes 1,3 wherein the conductive element is also adhesive the inventors propose the following equations (3) and (4). In case of dry electrodes wherein the conductive element is not adhesive, equations (1) and (2) may be used instead.

$$\text{Required adhesion force}_{dry} = [\text{Peel force}_{skin\ adhesive} \times (\text{second surface area - surface area}_{cond.\ element})] + (\text{Peel force}_{cond.}\ \text{element} \times \text{Surface area}_{cond.}\ \text{element}) \tag{3},$$

Wherein the required adhesion force is the target adhesion force of adhesive layer 30 at the second surface 46; Peel force$_{skin\ adhesive}$ is the peel force of the adhesive material at the second surface 46 covered by the adhesive, excluding the conductive element 10; Peel force$_{cond.\ element}$ is the peel force of the adhesive material of the conductive element 10; Surface area$_{cond.\ element}$ is the area covered by the surface of the conductive element 10.

Thus, in case of a dry electrode 1, 3, having a conductive element 10 being also adhesive, adhesive layer 30 comprises two parts: a first adhesive layer part which at least partly surrounds the conductive element 10, and for example is made of acrylic, silicone, or rubber and a second adhesive layer part, which is the adhesive layer of the conductive element 10.

Thus, the area of the second surface 46 for a dry electrode 1, 3 can be estimated according to equation (3). The required adhesion force$_{ary}$ is chosen based on the use case of the electrode 1, 3, as for example discussed above. Thus, knowing the peel force properties of the skin adhesive and the conductive element, the required adhesion force$_{dry,}$ and the surface area of the conductive element 10, the area of the second surface 46 can be calculated.

As discussed above the peel force may be provided as force per width of the material (instead of force per surface area). Thus, in such cases the following equation (4) can be used for dry electrodes.

$$\text{Required adhesion force}_{dry} = [(\text{Peel force}_{skin\ adhesive} \times (\text{max width}_{second\ surface\ area} \text{ - max width conductive element})] + (\text{Peel force}_{cond.}\ \text{element} \times \text{max width}_{cond.\ element}) \tag{4},$$

Wherein the required adhesion force is the target adhesion force of adhesive layer 30 at the second surface 46; Peel force$_{skin\ adhesive}$ is the peel force of the adhesive material at the second surface 46 covered by the adhesive, excluding the surface of the conductive element 10; Peel force$_{cond.\ element}$ is the peel force of the adhesive material of the conductive element 10, max width$_{second\ surface\ area}$ is the maximum width of the second surface 46, at a direction perpendicular to the direction of the peel force, e.g., for a circular electrode it is the diameter of the second surface 46, and for a square electrode it is its side. Max width $_{conductive\ element}$ is the maximum width of the conductive element, at a direction perpendicular to the direction of the peel force, e.g., for a circular conductive element it is the diameter of the conductive element.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

**Claims**

1.  An electrode (1) for physiological measurements, the electrode comprising:

    a conductive element (10) for acquiring a biosignal from a subject;
    a connector (20) for connecting to a biosignal acquisition device;
    an adhesive layer (30) for adhering the electrode to the subject; and
    a cutting line (40, 40a, 40b);
    wherein the adhesive layer defines a first outer edge (35) of a first surface (36) of the electrode;
    wherein the cutting line at least partly defines a second outer edge (45) of a second surface (46) of the electrode;
    wherein the second surface is a subsection of the first surface; and
    wherein the second surface is arranged to transfer the biosignal from the subject via the conductive element to the connector.

2. The electrode of claim 1, wherein the electrode comprises two or more different cutting lines.

3. The electrode of any of the preceding claims, wherein the electrode has a first side (50), and a second side (60) opposing the first side, the first side comprising the adhesive layer;
   wherein the cutting line is located at the first and/or at the second side.

4. The electrode of any of the preceding claims, wherein the cutting line is located on a template layer, and wherein the template layer is attached on the electrode.

5. The electrode of any of the preceding claims, wherein the cutting line outlines the shape of at least one of a circle, an ellipse, a hexagon and a square.

6. A device (2) adapted to trim an electrode (3) for physiological measurements,
   wherein the electrode (1,3) comprises:

   a conductive element (10) for acquiring a biosignal from a subject;
   a connector for connecting to a biosignal acquisition device; and
   an adhesive layer for adhering the electrode to the subject, wherein the adhesive layer defines a first outer edge (35) of a first surface (36) of the electrode;

   the device comprising:

   a holder (110) for holding the electrode, and;
   a trimming mechanism (120) adapted to trim the electrode along a cutting line (40, 40a, 40b);
   wherein the cutting line at least partly defines a second outer edge (45) of a second surface (46) of the electrode;
   wherein the second surface is a subsection of the first surface; and
   wherein the second surface is arranged to transfer the biosignal from the subject via the conductive element to the connector.

7. The device of claim 6, wherein the trimming mechanism is configurable to trim the electrode along two or more different cutting lines.

8. The device of claims 6 or 7, wherein the cutting line outlines the shape of at least one of a circle, an ellipse, a hexagon and a square.

9. The device of claims 6-8, adapted to trim an electrode (1) according to claims 1-5.

10. The device of claims 6-9, wherein the holder comprises the trimming mechanism.

40

20

60

1 →

30

10

50

FIG. 1

1 →

36

35

50

30

10

40a

40b

45

46

FIG. 2

1 →

60

20

40a

40b

46

FIG. 3

FIG. 4

FIG. 5

FIG. 6

FIG. 7

Europäisches
Patentamt
European
Patent Office
Office européen
des brevets

# EUROPEAN SEARCH REPORT

**Application Number**

EP 24 19 8491

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Philips: "A fetal monitoring solution designed around you",<br><br>, 23 May 2017 (2017-05-23), XP093242464, Retrieved from the Internet: URL:chrome-extension://efaidnbmnnnibpcajpc glclefindmkaj/https://www.documents.philip s.com/assets/20170523/645794384ea545e5ab83 a77c0158ebc1.pdf<br>* page 2: attachment electrode 989803139771 * | 1-5 | INV.<br>A61B5/257<br>A61B5/28<br>A61B5/00 |
| X | US 6 240 323 B1 (CALENZO SR JAMES C [US] ET AL) 29 May 2001 (2001-05-29)<br>* column 3, line 59 - column 4, line 39; figures 1-6 * | 1-5 | |
| X | US 8 165 655 B2 (GRASSL THOMAS [DE]; DRAEGER MEDICAL GMBH [DE]) 24 April 2012 (2012-04-24)<br>* figures 1-5 * | 1-5 | |
| | | | TECHNICAL FIELDS SEARCHED (IPC) |
| X | CN 214 434 233 U (UNIV SICHUAN WEST CHINA 2ND UNIV HOSPITAL) 22 October 2021 (2021-10-22)<br>* figures 1-4 * | 1-5 | A61B |
| X | US 2002/007713 A1 (LEE CHENG-CHEN [TW]) 24 January 2002 (2002-01-24)<br>* figure 1 * | 6-10 | |
| X | KR 2015 0119612 A (CHOE JAE YONG [KR]) 26 October 2015 (2015-10-26)<br>* figures 1,2 * | 6-10 | |

The present search report has been drawn up for all claims

| Place of search | Date of completion of the search | Examiner |
|---|---|---|
| The Hague | 23 January 2025 | Lommel, André |

## ANNEX TO THE EUROPEAN SEARCH REPORT
## ON EUROPEAN PATENT APPLICATION NO.

EP 24 19 8491

This annex lists the patent family members relating to the patent documents cited in the above-mentioned European search report.
The members are as contained in the European Patent Office EDP file on
The European Patent Office is in no way liable for these particulars which are merely given for the purpose of information.

23-01-2025

| Patent document cited in search report | | Publication date | Patent family member(s) | | Publication date |
|---|---|---|---|---|---|
| US 6240323 | B1 | 29-05-2001 | AT | E253955 T1 | 15-11-2003 |
| | | | DE | 69912802 T2 | 12-08-2004 |
| | | | EP | 1104325 A1 | 06-06-2001 |
| | | | JP | 4155709 B2 | 24-09-2008 |
| | | | JP | 2002522178 A | 23-07-2002 |
| | | | US | 6240323 B1 | 29-05-2001 |
| | | | WO | 0009202 A1 | 24-02-2000 |
| US 8165655 | B2 | 24-04-2012 | CN | 101385646 A | 18-03-2009 |
| | | | DE | 102007044019 A1 | 09-04-2009 |
| | | | EP | 2036493 A1 | 18-03-2009 |
| | | | US | 2009076365 A1 | 19-03-2009 |
| CN 214434233 | U | 22-10-2021 | NONE | | |
| US 2002007713 | A1 | 24-01-2002 | DE | 10134523 A1 | 21-03-2002 |
| | | | TW | 542079 U | 11-07-2003 |
| | | | US | 2002007713 A1 | 24-01-2002 |
| KR 20150119612 | A | 26-10-2015 | NONE | | |

EPO FORM P0459

For more details about this annex : see Official Journal of the European Patent Office, No. 12/82